# EUROPEAN PATENT SPECIFICATION

(11) **EP 1 427 344 B1**
(45) Date of publication and mention of the grant of the patent: **24.11.2010**
(21) Application number: 02737282.0
(22) Date of filing: 31.05.2002
(51) Int. Cl.: A61B 18/02

(54) **CRYOGENIC SYSTEM**
KRYOGENES SYSTEM
SYSTEME CRYOGENIQUE

(30) Priority: 31.05.2001 US 294256 P
(43) Date of publication of application: 16.06.2004
(73) Proprietor: Endocare, Inc., Irvine, CA 92618 (US)
(72) Inventor: BAUST, John, G., Candor, NY 13743 (US); CHEEKS, Roy, Harpers Ferry, WV 25425 (US)
(74) Representative: Wagner, Karl H.
(86) International application number: PCT/US2002/017104
(87) International publication number: WO 2002/096270

(56) References cited:
- US-A- 3 613 689
- US-A- 4 472 946
- US-A- 5 916 212
- US-A- 5 947 960
- US-A- 5 957 963

## Description

### Field of the Invention

The present invention relates to a cryogenic system. More specifically, illustrative embodiments of the present invention relate to a cryogenic system for use in cryosurgical procedures.

### Background

The distribution of boiling (liquid) cryogens, such as liquid nitrogen, is problematic due to the parasitic heat load provided by a cryosurgical device's plumbing or transport circuit, which is maintained at ambient temperature. Precooling the plumbing circuit, even if adequately insulated, causes two-phase flow (liquid-gas mixtures), cryogen boil-off, and choking flow due to gas expansion in the transport circuit. As a result, target temperatures at the distal end of the flow path (i.e., cryoprobe tip) are not reached for many minutes.

US-A-4,472,946 discloses a cryogenic storage tank with a built-in pump for pumping cryogen directly from the primary storage container consistent with low boil-off losses of cryogen. The cryogenic storage tank has an outer vessel, an inner vessel and an evacuated insulation space therebetween. A pump mounting tube assembly extends into the interior of the inner vessel and includes an inner pump mounting tube and an outer pump mounting tube joined at their lower rims to define an insulating jacket between the two tubes. The inner pump mounting tube is affixed at its upper end to the outer vessel while the outer pump mounting tube is affixed at its upper end to the inner vessel. The inner pump mounting tube defines a relatively long heat path into the cryogenic container and is itself insulated from the liquid cryogen by a pocket of trapped gas formed within the inner pump mounting tube by heated cryogen. A pump may be introduced through the inner pump mounting tube and is also insulated against contact with liquid cryogen by the trapped gas such that only the lowermost end of the pump is immersed in cryogen thereby minimizing heat leakage into the tank.

US-A-5,957,963 discloses a method and apparatus for performing hypothermia of a selected organ without significant effect on surrounding organs or other tissues. A flexible catheter is inserted through the vascular system of a patient to place the distal tip of the catheter in an artery feeding the selected organ. A compressed refrigerant is pumped through the catheter to an expansion element near the distal tip of the catheter, where the refrigerant vaporizes and expands to cool a flexible bellows shaped heat transfer element in the distal tip of the catheter. The heat transfer bellows cools the blood flowing through the artery, to cool the selected organ, distal to the tip of the catheter. In a preferred embodiment, the flexible catheter comprises a bellows shaped heat transfer element on the distal end of the catheter body. The heat transfer bellows can be constructed of a metal having a high thermal conductivity, such as nickel, copper, silver, gold, or some other suitable material. The heat transfer bellows comprises a tubular throat fitted within the distal section of the catheter. A plurality of annular folds extend from the distal end of the throat. An end cap is formed at the distal end of the annular folds. To prevent thrombosis, an antithrombogenic agent such as heparin can be bonded to the outer surface of the bellows, particularly on the annular folds. Alternatively, the outer surface of the bellows can be nitrided or subjected to a similar treatment to create a smooth surface which retards thrombosis.

### Summary

The present invention can be embodied in a pump assembly for a cryosurgical pump as set forth in claim 1.

The present invention may also be embodied in a cryosurgical system, as set forth in claim 2.

Also disclosed is a method of delivering cryogen to a surgical device, comprising providing a container having cryogen within the container; providing a pump having a piston within a cylinder and submerged within the cryogen; providing a surgical instrument outside the container for use in cryosurgical procedures; providing a system of conduits fluidly interconnecting the piston and the surgical instrument permitting the cryogen to be forced from the piston to the probe upon activation of the piston; providing a pressure relief device fluidly coupled to the systems of conduits and positioned between the bellows and the probe; activating the piston to pull cryogen within the cylinder; activating the piston to push the cryogen from the cylinder to the surgical instrument at an initial predetermined pressure.

Other aspects, features, and advantages of the present invention will become apparent from the following detailed description of the illustrated embodiments, the accompanying drawings, and the appended claims.

### Brief Description of the Drawings

FIG. 1 illustrates a system in accordance with an embodiment of the present invention and includes a cross-sectional view through a container holding cryogen;
FIG. 2 is an enlarged, elevational view of the container of FIG. 1 and its contents;
FIG. 3 is an enlarged, top view of the assembly within the container of FIG. 1;
FIG. 4 is a top view of a valve used in the system illustrated in FIGS. 1-3;
FIG. 5 is an elevational view of the valve of FIG. 4;
FIG. 6 is a schematic view of the system illustrated in FIG. 1 and showing the system as the bellows pulls cryogen within the cylinder;
FIG. 7 is a schematic view similar to FIG. 6 but showing the system as the bellows initially pushes cryogen to the probe; and
FIG. 8 is a schematic view similar to FIGS. 6 and 7 but showing the system as the bellows pushes cryogen to the probe and with the pressure relief valve activated to control the fluid pressure.

### Detailed Description of Illustrated Embodiments

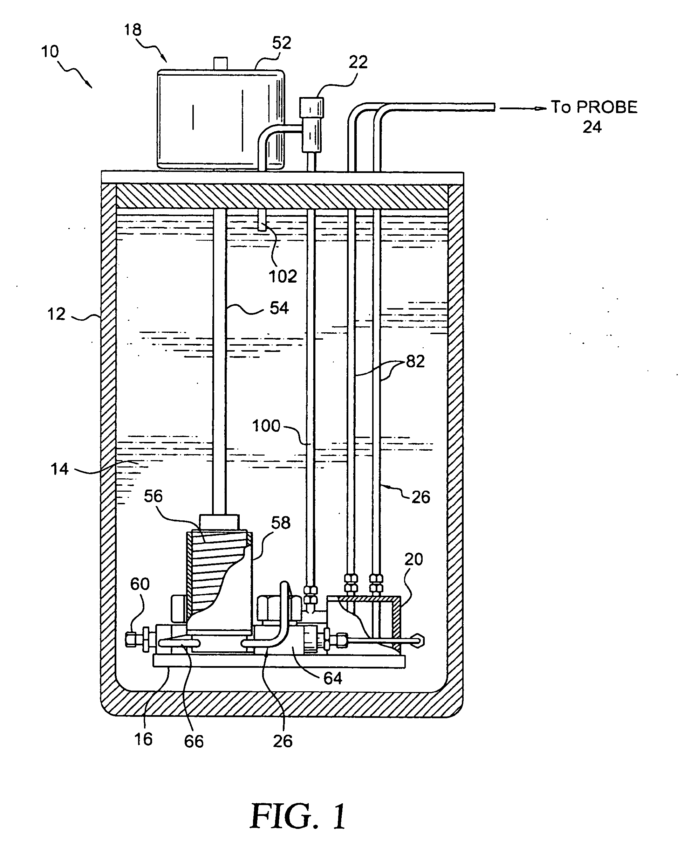

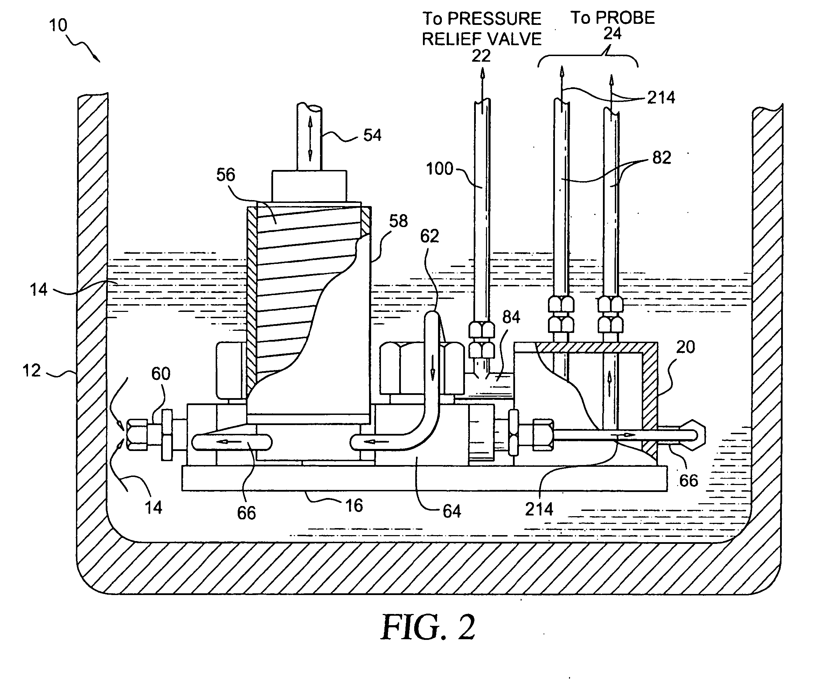

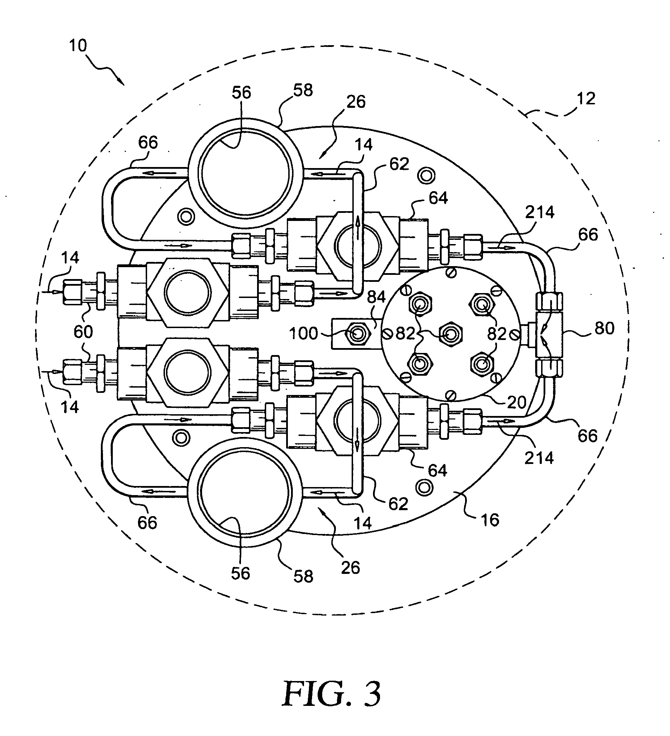

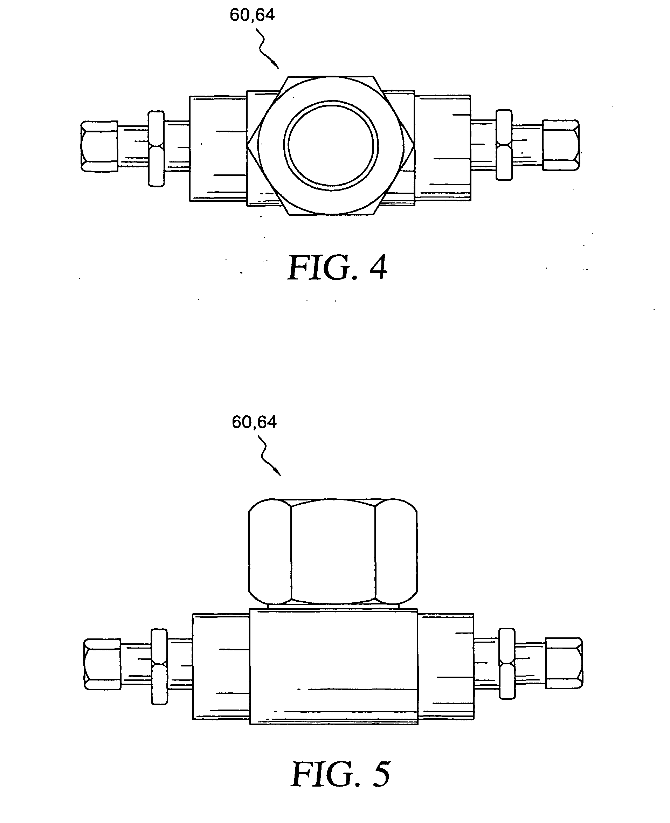

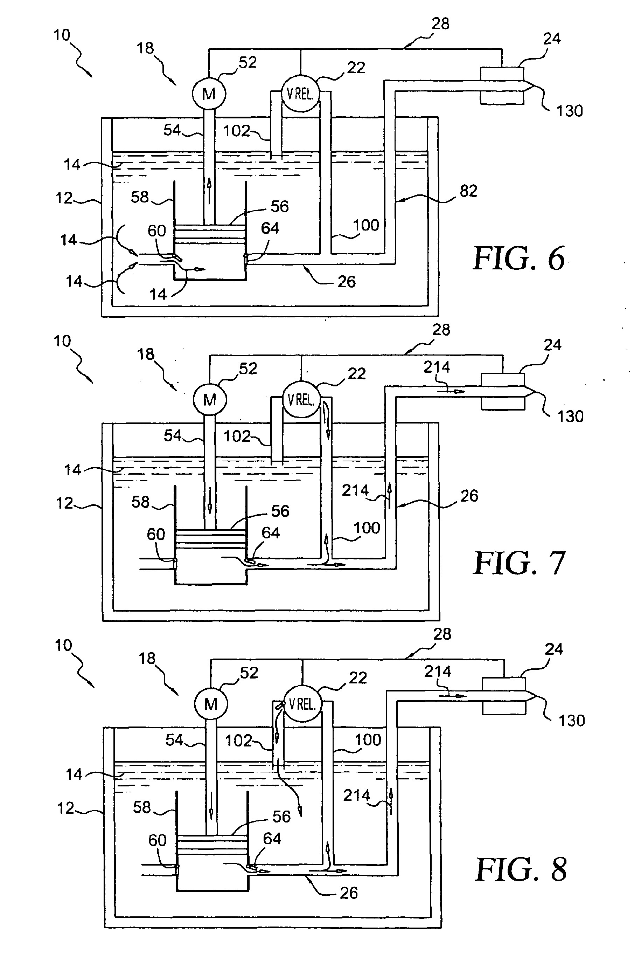

FIGS. 1-8 illustrate a preferred embodiment of a cryogenic pump and system according to the present invention. The system 10 provides instantaneous sub-cooling of liquid cryogen 14, thereby creating a cryogen state characterized by an excess capacity to absorb heat without boiling. By manipulating the pressure relationships in the plumbing circuit 26, sub-cooled liquid 214 is transported to the distal cryoprobe tip 130 in 1-20 seconds allowing near instantaneous freezing at the probe tip 130. This rate of cooling is faster and the attainable low temperature is lower than comparable Joule-Thompson-based cryogenic devices.

The illustrated system 10 includes a container or dewar 12 containing cryogen 14, such as liquid nitrogen. A support 16 is positioned within the container 12 and submerged within the cryogen 14. A pump 18 is coupled to the container 12 such that the piston 56, illustrated in the form of a bellows, is also submerged within the cryogen 14. By way of a conduit system 26, the bellows 56 is fluidly coupled to an output manifold 20, a pressure release valve 22, and a surgical probe 24. A control system 28 monitors the temperature of the probe tip 130 and adjusts the system components as necessary to maintain the desired temperature at probe tip 130.

The container 12 is of substantially conventional design, except that it is appropriately adapted to support pump 18. Pump 18 includes a linear drive motor 52 mounted outside the container 12 and, preferably mounted to the top of the container 12. A drive shaft 54 extends down from the motor 52, through the cryogen 14 and couples to the bellows 56. The bellows 56 is appropriately constructed to fit within a cylinder 58. Bellows 56 is preferably formed from stainless steel. Also, although the figures illustrate a system 10 with two bellows 56, any appropriate number of bellow systems may be employed. The cylinder 58 has a one-way inlet valve 60 coupled to an inlet conduit 62, and a one-way outlet valve 64 coupled to an outlet conduit. The cylinder 58 and bellows 56 assembly is rigidly secured to the support 16 along with manifold 20.

Manifold 20 has an inlet conduit 80 fluidly coupled to the outlet valves 64 of the bellows 56. Manifold 20 also has outlet conduits 82 fluidly coupled to the probe 24. Although five outlet conduits 82 are illustrated it should be understood that the number of outlet conduits 82 is dependent on the system requirements, and that more or fewer outlet conduits 82 may be used. The manifold 20 also has an outlet port 84 for coupling with the pressure relief valve 22.

Pressure relief valve 22 is preferably mounted outside the container 12 and is coupled to the manifold by inlet conduit 100. The valve 22 also has an outlet conduit that leads back into the cryogen 14 in container 12 to return, to the container 12, the cryogen 14 that has been released from the conduit system 26 to lower the pressure as desired.

The surgical apparatus illustrated is shown as probe 24 with its corresponding tip 130 and is intended to represent any appropriate cryosurgical device, including probe tips, such as those known in the prior art.

Thus, the illustrated embodiment includes a reciprocating stainless steel bellows pump 18 that operates while submerged in liquid cryogen 14 and that causes instantaneous sub-cooling of the liquid cryogen 14 during the compression stroke of the bellows 56, thereby creating a cryogen state characterized by an excess capacity to absorb heat without boiling. Further, by manipulating the pressure relationships in the plumbing circuit 26, sub-cooled liquid 214 is transported to the distal cryoprobe tip 130 in 1-20 seconds allowing near instantaneous freezing at the probe tip 130.

In the illustrated embodiment, one or more stainless steel bellows "pistons" 56 are driven by a surface mounted linear drive system 52 capable of pressure regulation. The reciprocating action of each bellows piston 56 a) sequentially produces a negative pressure to draw in liquid cryogen 14 on the fill stroke through a one-way check valve 60 and b) sequentially discharge liquid cryogen 214 at a prescribe pressure profile out through a second one-way check valve 64 to a pressure manifold 20 connected to the probe 24 plumbing circuitry. The prescribed profile is a range of between 1.724 MPa (250 pounds per square inch (psi)) and 2.758 MPa (400 pounds per square inch (psi)).

The pressure pulse profile establishes an initial high, transient pressure spike that causes a colligative-based sub-cooling of the liquid cryogen 14, which establishes a boiling point differential of approximately 30-40 degrees Celsius, thereby establishing an excess temperature capacity (the level of temperature rise that can be allowed before boiling of the cryogen 14) supporting the instantaneous distribution of sub-cooled cryogen 214 in the plumbing circuit 26. Following the transient pressure spike, pressure is reduced to a base level to sustain the desired rate of ice growth a the distal end of the circuit, that is, at the probe 24. Conventional control system technology can be employed in controlling the interaction between the probe 24 and the pressure relief valve 22 and/or the pump 18, that is, in producing the control system 28

Pressure relief and control is provided by an appropriately designed pressure transducer-valve interface 22 outside the cryogen-containing dewar 12.

## Claims

1. A pump assembly (18) for a cryosurgical system, comprising:
a driving mechanism (52) coupled to an elongated drive shaft (52);
a bellows (56) coupled to said drive shaft (52) and adapted to be submersed in cryogen, said bellows (56) formed from metal;
a one-way inlet valve (60) fluidly coupled to said bellows (56);
a one-way outlet valve (64) fluidly coupled to said bellows (56); and
a supply manifold (20) fluidly coupled with said outlet valve (64), said supply manifold (20) having a plurality of outlet ports (82) for connection to said cryoprobe (24),
wherein said pump assembly (18) provides a pressure to said cryoprobe (24) in a range between 1,724 MPa (250 pounds per square inch) and 2.758 MPa (400 pounds per square inch).

2. A cryosurgical system (10), comprising:
a container (12) having cryogen (14) within said container (12);
a pump assembly as set forth in claim 1, having said bellows (56) submerged within said cryogen (14);
a cryoprobe (24) outside the container (12) for use in cryosurgical procedures;
a system of conduits (26) fluidly interconnecting said bellows (56) and said cryoprobe (24) permitting said cryogen (14) to be forced from said bellows (56) to said cryoprobe (24) upon activation of said bellows (56); and
a pressure relief device (22) fluidly coupled to said system of conduits (26) and positioned between said bellows (56) and said cryoprobe (24).

3. A system according to claim 2, wherein said cryogen (14) is liquid nitrogen.

## Patentansprüche

1. Pumpanordnung (18) für ein cryochirurgisches System, das Folgendes aufweist:
einen Antriebsmechanismus (52), der an eine langgestreckte Antriebswelle (52) angeschlossen ist;
ein Federbalg (56), der an die Antriebswelle (52) gekoppelt ist und
geeignet ist in einem Cryogen untergetaucht zu sein, wobei der Federbalg (56) aus Metall gebildet ist;
ein Ein-Wege-Einlassventil (60), das strömungsmäßig mit dem Federbalg (56) verbunden ist;
ein Ein-Wege-Auslassventil (64), das strömungsmäßig mit dem Federbalg (56) verbunden ist; und
ein Lieferverteiler (20), der strömungsmäßig mit dem Auslassventil (64) verbunden ist, wobei der Lieferverteiler (20) eine Vielzahl von Auslassanschlüssen (82) zur Verbindung mit dem Kältefühler (24) aufweist,
wobei die Pumpanordnung (18) einen Druck an den Kältefühler (24) in einem Bereich von 1,724 MPa (250 Pfund pro Quadrazoll) bis 2,758 MPa (400 Pfund pro Quadrazoll) bereitstellt.

2. Cryochirurgisches System (10), das Folgendes aufweist:
einen Behälter (12) mit Cryogen (14) in dem Behälter (12);
eine Pumpanordnung gemäß Anspruch 1, wobei der Federbalg (56) in dem Cryogen (14) untergetaucht ist;
ein Kältefühler (24) außerhalb des Behälters (12) zur Verwendung in cryochirurgischen Anwendungen;
ein System von Leitungen (26), das strömungsmäßig den Federbalg (56) und den Kältefühler (24) verbindet, und erlaubt, dass das Cryogen (14) von dem Federbalg (56) zu dem Kältefühler (24) beim Aktivieren des Federbalgs (56) gedrückt wird; und
eine Druckentlastungsvorrichtung (52), die strömungsmäßig mit dem System von Leitungen (26) verbunden ist und zwischen dem Federbalg (56) und dem Kältefühler (24) angeordnet ist.

3. System gemäß Anspruch 2, wobei das Cryogen (14) flüssiger Stickstoff ist.

## Revendications

1. Ensemble de pompe (18) pour un système cryogénique chirurgical, comprenant :
un mécanisme d'entraînement (52) couplé à un arbre d'entraînement allongé (52) ;
un soufflet (56) couplé à l'arbre d'entraînement (52) et adapté à être immergé dans un liquide cryogénique, ledit soufflet (56) étant en métal ;
une soupape d'entrée monodirectionnelle (60) en liaison de fluide avec le soufflet (56) ;
une soupape de sortie monodirectionnelle (64) en liaison de fluide avec le soufflet (56) ; et
un distributeur d'alimentation (20) en liaison de fluide avec la soupape de sortie (64), le distributeur d'alimentation (20) comportant une pluralité d'accès de sortie (82) pour connexion à la sonde cryogénique (24),
l'ensemble de pompe (18) fournissant une pression à la sonde cryogénique (24) dans une plage comprise entre 1724 MPa (250 livres par pouce carré) et 2758 MPa (400 livres par pouce carré).

2. Système cryogénique chirurgical (10), comprenant :
un conteneur (12) contenant un liquide cryogénique (14) dans le conteneur (12) ;
un ensemble de pompe selon la revendication 1, ayant le soufflet (56) immergé dans le liquide cryogénique (14) ;
une sonde cryogénique (24) à l'extérieur du conteneur (12) pour utilisation dans des procédures de chirurgie cryogénique ;
un système de conduits (26) interconnectant un fluide entre le soufflet (56) et la sonde cryogénique (24) ce qui permet au liquide cryogénique (14) d'être forcé à partir du soufflet (56) vers la sonde cryogénique (24) à l'actionnement du soufflet (56) ; et
un dispositif de décharge de pression (22) en liaison de fluide avec le système de conduits (26) et disposé entre le soufflet (56) et la sonde cryogénique (24).

3. Système selon la revendication 2, dans lequel le liquide cryogénique (14) est de l'azote liquide.
